# EUROPEAN PATENT SPECIFICATION

(11) **EP 2 370 076 B1**
(45) Date of publication and mention of the grant of the patent: **04.01.2017**
(21) Application number: 09756528.7
(22) Date of filing: 25.11.2009
(51) Int. Cl.: A61K 31/436, A61K 31/5377, A61K 45/06, A61P 35/00

(54) **Pharmaceutical combination comprising a Hsp 90 inhibitor and a mTOR inhibitor**
Pharmazeutische Zusammensetzung enthaltend einen Hsp 90-Inhibitor und einen mTOR-Inhibitor
Combinaison pharmaceutique comprenant un inhibiteur de Hsp 90 et un inhibiteur de mTOR

(30) Priority: 28.11.2008 EP 08170279; 28.11.2008 EP 08170287; 28.11.2008 EP 08170230; 28.11.2008 EP 08170246
(43) Date of publication of application: 05.10.2011
(73) Proprietor: Novartis AG, 4056 Basel (CH)
(72) Inventor: JENSEN, Michael, Rugaard, CH-4002 Basel (CH); GARCIA-ECHEVERRIA, Carlos, 4002 Basel (CH)
(74) Representative: Roth, Peter Richard
(86) International application number: PCT/EP2009/065858
(87) International publication number: WO 2010/060937

(56) References cited:
- WO-A1-2004/072051
- WO-A1-2007/041362
- WO-A2-2004/108080
- LANG S A; MOSER C; POPP F C; DAHLKE M H; GAUMANN A; OBED AIMAN; SCHLITT H J; GEISSLER E K; STOELTZING O: "Simultaneous inhibition of Hsp90 and mTOR synergistically reduces tumor growth and angiogenesis of hepatocellular cancer in an experimental model" PROCEEDINGS OF THE ANNUAL MEETING OF THE AMERICAN ASSOCIATION FOR CANCER RESEARCH, AMERICAN ASSOCIATION FOR CANCER RESEARCH, US, vol. 48, 1 April 2007 (2007-04-01), page 506, XP001537125 ISSN: 0197-016X
- FRANCIS LANIE K ET AL: "Combination mammalian target of rapamycin inhibitor rapamycin and HSP90 inhibitor 17-allylamino-17-demethoxygeldanamycin has synergistic activity in multiple myeloma" CLINICAL CANCER RESEARCH, THE AMERICAN ASSOCIATION FOR CANCER RESEARCH, US, vol. 12, no. 22, 15 November 2006 (2006-11-15), pages 6826-6835, XP002522331 ISSN: 1078-0432
- ALSAYED YAZAN ET AL: "Combination of the mTOR inhibitor rapamycin with the HSP90 inhibitor 17-AAG has synergistic activity in EBV positive post transplant lymphoproliferative disorders (PTLD)" BLOOD, AMERICAN SOCIETY OF HEMATOLOGY, US, vol. 106, no. 11, Part 1, 1 November 2005 (2005-11-01), page 679A, XP009114887 ISSN: 0006-4971

## Description

### BACKGROUND OF THE INVENTION

### Field of the Invention

The present invention is directed to a pharmaceutical composition comprising an Hsp90 inhibitor and an mTOR inhibitor, as defined in the claims, and the uses of such a composition for the treatment of proliferative diseases, more specifically of mammalian target of rapamycin (mTOR) kinase and dependent diseases.

In spite of numerous treatment options for proliferative disease patients, there remains a need for effective and safe antiproliferative agents and a need for their use in combination therapy.

It has now been found that a combination comprising at least one Hsp90 inhibitor compound and at least one mTOR inhibitor, as defined below, has a beneficial effect on proliferative disorders, including without limitation, solid tumors, myelomas, leukemias, psoriasis, restenosis, sclerodermitis and fibrosis.

### Description of Related Art

Heat shock protein 90 (Hsp90) is recognized as an anti-cancer target. Hsp90 is a ubiquitous, highly abundant (1-2% of the total cellular protein), essential protein which functions as a molecular chaperone to ensure the conformational stability, shape and function of client proteins.

Among the stress proteins, Hsp90 is unique because it is not required for the biogenesis of most polypeptides (Nathan et al., 1997). Its cellular targets, also called client proteins, are conformationally labile signal transducers that play a critical role in growth control, cell survival and tissue development (Pratt and Toft, 2003). Inhibition of its intrinsic ATPase activity of Hsp90 disrupts the Hsp90-client protein interaction resulting in their degradation via the ubiquitin proteasome pathway. A subset of Hsp90 client proteins, such as Raf, AKT, phospho-AKT, CDK4 and the EGFR family including ErbB2 are oncogenic signaling molecules critically involved in cell growth, differentiation and apoptosis, processes which are important in cancer cells. The degradation of one or multiple oncoproteins is believed to produce the anti-tumor effects observed with Hsp90 inhibitors.

The Hsp90 family of chaperones is comprised of four members: Hsp90α and Hsp90β both located in the cytosol, GRP94 in the endoplasmic reticulum, and TRAP1 in the mitochondria (Csermely et al., 1998). Hsp90 is the most abundant cellular chaperone, constituting about 1% - 2% of total protein (Jakob and Buchner, 1994).

Hsp90 chaperones, which possess a conserved ATP-binding site at their N-terminal domain (Chene, 2002) belong to a small ATPase sub-family known as the DNA Gyrase, Hsp90, Histidine Kinase and MutL (GHKL) sub-family (Dutta and Inouye, 2000). The chaperoning (folding) activity of Hsp90 depends on its ATPase activity which is weak for the isolated enzyme. However, it has been shown that the ATPase activity of Hsp90 is enhanced upon its association with proteins known as co-chaperones (Kamal et al., 2003). Therefore, *in vivo,* Hsp90 proteins work as subunits of large, dynamic protein complexes. Hsp90 is essential for eukaryotic cell survival and is overexpressed in many tumors.

Treatment of cancer cells with an mTOR inhibitor can cause up-regulation of the pro-survival protein phospho-AKT (O'Reilly, 2006). Since phospho-AKT is an Hsp90 client protein, co-treatment of with an Hsp90 inhibitor would prevent or diminish the mTOR inhibitor induced upregulation of phospho-AKT giving rise to an increased anti-tumor effect.

### SUMMARY OF THE INVENTION

The present invention also relates to the use of a Hsp90 inhibitor which is 5-(2,4-dihydroxy-5-isopropyl-phenyl)-4-(4-morpholin-4-ylmethyl-phenyl)-isoxazole-3-carboxylic acid ethylamide, or a pharmaceutically acceptable salt thereof.

Combinations of the present disclosure include compounds which target, decrease or inhibit the activity/function of serine/theronine mTOR kinase. Such compounds will be referred to as "mTOR inhibitors" and include but is not limited to compounds, proteins or antibodies which target/inhibit members of the mTOR kinase family, e.g., RAD, rapamycin (sirolimus) and derivatives/analogs thereof such as everolimus or RAD001. Sirolimus is also known by the name RAPAMUNE and everolimus or RAD001 by the name CERTICAN. Other compounds, proteins or antibodies which target/inhibit members of the mTOR kinase family include CCI-779, ABT578, SAR543, and ascomycin which is an ethyl analog of FK506. Also included are AP23573 and AP23841 from Ariad.

Preferred mTOR inhibitors are everolimus, rapamycin, ascomycin and rapamycin derivatives.

### SUMMARY OF THE DRAWINGS

Fig. 1 shows the Akt phosphorylation levels in presence of everolimus (RAD001) and everolimus (RAD001) in combination with compound I in BT474 breast tumor cells.
Fig. 2 shows the AKT phosphorylation levels in presence of everolimus (RAD001) and everolimus (RAD001) in combination with compound I in MDA-MB-231 breast tumor cells.

### DETAILED DESCRIPTION OF THE INVENTION

The following definitions are provided to better understand the invention.

As used herein, the term "pharmaceutically acceptable salts" refers to the nontoxic acid or alkaline earth metal salts of the 2-amino-7,8-dihydro-6H-pyrido[4,3-d]pyrimidin-5-one compounds of the disclosure. These salts can be prepared *in situ* during the final isolation and purification of the 2-amino-7,8-dihydro-6H-pyrido[4,3-d]pyrimidin-5-one compounds, or by separately reacting the base or acid functions with a suitable organic or inorganic acid or base, respectively. Representative salts include, but are not limited to, the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemi-sulfate, heptanoate, hexanoate, fumarate, hydrochloride, hydrobromide, hydroiodide, 2-hydroxyethanesulfonate, lactate, maleate, methanesulfonate, nicotinate, 2-napthalenesulfonate, oxalate, pamoate, pectinate, persulfate, 3-phenylproionate, picrate, pivalate, propionate, succinate, sulfate, tartrate, thiocyanate, p-toluenesulfonate, and undecanoate. Also, the basic nitrogen-containing groups can be quaternized with such agents as alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl, and stearyl chlorides, bromides and iodides, aralkyl halides like benzyl and phenethyl bromides, and others. Water or oil-soluble or dispersible products are thereby obtained.

Examples of acids that may be employed to form pharmaceutically acceptable acid addition salts include such inorganic acids as hydrochloric acid, sulfuric acid and phosphoric acid and such organic acids as oxalic acid, maleic acid, methanesulfonic acid, succinic acid and citric acid. Basic addition salts can be prepared *in situ* during the final isolation and purification of the 2-amino-7,8-dihydro-6H-pyrido[4,3-d]pyrimidin-5-one compounds, or separately by reacting carboxylic acid moieties with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine, and the like.

The term "pharmaceutically acceptable prodrugs" as used herein refers to those prodrugs of the compounds of the present disclosure which are, within the scope of sound medical judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds of the disclosure. The term "prodrug" refers to compounds that are rapidly transformed *in vivo* to yield the parent compound of the above formula, for example by hydrolysis in blood, such an ester. A thorough discussion is provided in Higuchi, T., and V. Stella, "Pro-drugs as Novel Delivery Systems," A.C.S. Symposium Series 14, and in "Bioreversible Carriers in Drug Design," in Edward B. Roche (ed.), American Pharmaceutical Association, Pergamon Press, 1987, both of which are incorporated herein by reference.

The Hsp90 inhibitor of the pharmaceutical combination of the present invention is 5-(2,4-Dihydroxy-5-isopropyl-phenyl)-4-(4-morpholin-4-ylmethyl-phenyl)-isoxazole-3-carboxylic acid ethylamide, which is disclosed in WO 04/072051 published on August 26, 2004.

Suitable mTOR inhibitors include for example:
I. Rapamycin which is an immunosuppressive lactam macrolide that is produced by *Streptomyces hygroscopicus.*
II. Rapamycin derivatives such as:
   a. substituted rapamycin e.g. a 40-O-substituted rapamycin e.g. as described in US 5,258,389, WO 94/09010, WO 92/05179, US 5,118,677, US 5,118,678, US 5,100,883, US 5,151,413, US 5,120,842, WO 93/11130, WO 94/02136, WO 94/02485 and WO 95/14023;
   b. a 16-O-substituted rapamycin, such as disclosed in WO 94/02136, WO 95/16691 and WO 96/41807;
   c. a 32-hydrogenated rapamycin, such as described in WO 96/41807 and US 5,256,790.
   d. Preferred rapamycin derivatives are compounds of formula I' 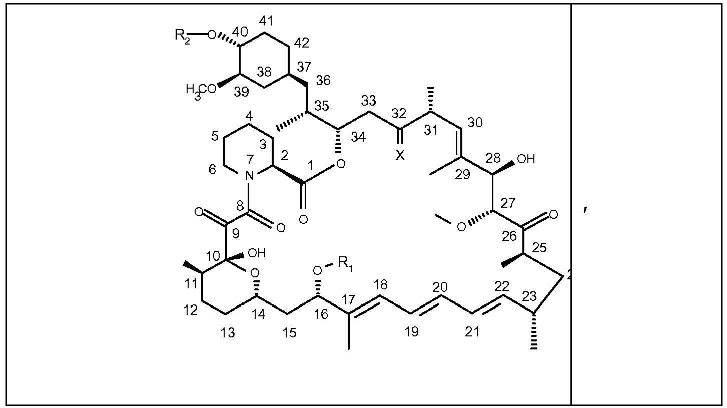 wherein
      R₁ is CH₃ or C₃₋₆alkynyl,
      R₂ is H or -CH₂-CH₂-OH, 3-hydroxy-2-(hydroxymethyl)-2-methyl-propanoyl or tetrazolyl, and X is =O, (H,H) or (H,OH)
      provided that R₂ is other than H when X is =O and R₁ is CH₃,
      or a prodrug thereof when R₂ is -CH₂-CH₂-OH, e.g. a physiologically hydrolysable ether thereof.

      Compounds of formula I' are disclosed, for example, in WO 94/09010, WO 95/16691 or WO 96/41807. They may be prepared as disclosed or by analogy to the procedures described in these references
      Preferred mTOR inhibitor compounds are 32-deoxorapamycin, 16-pent-2-ynyloxy-32-deoxorapamycin, 16-pent-2-ynyloxy-32(S)-dihydro-rapamycin, 16-pent-2-ynyloxy-32(S)-dihydro-40-O-(2-hydroxyethyl)-rapamycin and, more preferably, 40-O-(2-hydroxyethyl)-rapamycin, disclosed as Example 8 in WO 94/09010.
      Particularly preferred rapamycin derivatives of formula I' are 40-O-(2-hydroxyethyl)-rapamycin, 40-[3-hydroxy-2-(hydroxymethyl)-2-methylpropanoate]-rapamycin (also called CCl779), 40-epi-(tetrazolyl)-rapamycin (also called ABT578), 32-deoxorapamycin, 16-pent-2-ynyloxy-32(S)-dihydro rapamycin, or TAFA-93.
   e. Rapamycin derivatives also include so-called rapalogs, e.g. as disclosed in WO 98/02441 and WO 01/14387, e.g. AP23573, AP23464, or AP23841.
      Rapamycin and derivatives thereof have, on the basis of observed activity, e.g. binding to macrophilin-12 (also known as FK-506 binding protein or FKBP-12), e.g. as described in WO 94/09010, WO 95/16691 or WO 96/41807, been found to be useful e.g. as immunosuppressant, e.g. in the treatment of acute allograft rejection.
      The present invention provides:
      a pharmaceutical combination comprising:
         5-(2,4-Dihydroxy-5-isopropyl-phenyl)-4-(4-morpholin-4-ylmethyl-phenyl)-isoxazole-3-carboxylic acid ethylamide, or a pharmaceutically acceptable salt thereof; and
      b) at least one mTOR inhibitor, as defined in the claims.
         In a further aspect the present invention provides a compound which is 5-(2,4-Dihydroxy-5-isopropyl-phenyl)-4-(4-morpholin-4-ylmethyl-phenyl)-isoxazole-3-carboxylic acid ethylamide, or a pharmaceutically acceptable salt thereof, and at least one mTOR inhibitor, as defined in the claims, for use in treating or preventing a proliferative disease.
         The mTOR inhibitor used according to the present invention may be selected from RAD rapamycin (sirolimus) and derivatives/analogs thereof such as everolimus or RAD001; CCI-779, AP23573. Particularly preferred mTOR inhibitors in accordance with the present invention are sirolimus and/or everolimus.
III. Disclsosed are likewise the pharmaceutically acceptable salts thereof, the corresponding racemates, diastereoisomers, enantiomers, tautomers, as well as the corresponding crystal modifications of above disclosed compounds where present, e.g. solvates, hydrates and polymorphs, which are disclosed therein. The compounds used as active ingredients in the combinations of the invention can be prepared and administered as described in the cited documents, respectively. Also within the scope of this invention is the combination of more than two separate active ingredients as set forth above, i.e., a pharmaceutical combination within the scope of this invention could include three active ingredients or more.

In embodiments, the mTOR inhibitor used according to the present invention may be selected from RAD rapamycin (sirolimus) and derivatives/analogs thereof such as everolimus or RAD001; CCI-779, ABT578, and AP23573.

The term "mTOR kinase dependent diseases" includes but is not restricted to the following symptoms:
- Organ or tissue transplant rejection, e.g. for the treatment of recipients of e.g. heart, lung, combined heart-lung, liver, kidney, pancreatic, skin or corneal transplants; graft-versus-host disease, such as following bone marrow transplantation;
- Restenosis
- Hamartoma syndromes, such as tuberous sclerosis or Ccowden Disease
- Lymphangioleiomyomatosis
- Retinitis pigmentosis
- Autoimmune diseases including encephalomyelitis, insulin-dependent diabetes mellitus, lupus, dermatomyositis, arthritis and rheumatic diseases
- Steroid-resistant acute Lymphoblastic Leukaemia
- Fibrotic diseases including scleroderma, pulmonary fibrosis, renal fibrosis, cystic fibrosis
- Pulmonary hypertension
- Immunomodulation
- Multiple sclerosis
- VHL syndrome
- Carney complex
- Familial adenonamtous polyposis
- Juvenile polyposis syndrome
- Birt-Hogg-Duke syndrome
- Familial hypertrophic cardiomyopathy
- Wolf-Parkinson-White syndrome
- Neurodegenarative disorders such as Parkinson's, Huntingtin's, Alzheimer's and dementias caused by tau mutations, spinocerebellar ataxia type 3, motor neuron disease caused by SOD1 mutations, neuronal ceroid lipofucinoses/Batten disease (pediatric neurodegeneration)
- wet and dry macular degeneration
- muscle wasting (atrophy, cachexia) and myopathies such as Danon's disease.
- bacterial and viral infections including M. tuberculosis, group A streptococcus, HSV type I, HIV infection
- Neurofibromatosis including Neurofibromatosis type 1,
- Peutz-Jeghers syndrome

Furthermore, "mTOR kinase dependent diseases" include cancers and other related malignancies. A non-limiting list of the cancers associated with pathological mTOR signaling cascades includes breast cancer, renal cell carcinoma, gastric tumors, neuroendocrine tumors, lymphomas and prostate cancer.

Examples for a proliferative disease the can be treated with a combination of Hsp90 inhibitor and mTOR, as defined in the claims, are for instance benign or malignant tumor, carcinoma of the brain, kidney, liver, adrenal gland, bladder, breast, stomach, gastric tumors, ovaries, colon, rectum, prostate, pancreas, lung, vagina or thyroid, genitourinary area, melanoma, glioma, sarcoma, glioblastomas, multiple myeloma or gastrointestinal cancer, especially colon carcinoma or colorectal adenoma or a tumor of the neck and head, an epidermal hyperproliferation, psoriasis, prostate hyperplasia, a neoplasia, a neoplasia of epithelial character, neuroblastoma, lymphomas, a mammary carcinoma, or a leukemia.

In particular, the inventive compositions are particularly useful for treating:
a breast tumor; an epidermoid tumor, such as an epidermoid head and/or neck tumor or a mouth tumor; a lung tumor, e.g., a small cell or non-small cell lung tumor; a gastrointestinal tumor, e.g., a colorectal tumor; or a genitourinary tumor, e.g., a prostate tumor (especially a hormone-refractory prostate tumor); or
   (ii) a proliferative disease that is refractory to the treatment with other chemotherapeutics; or
   (iii) a tumor that is refractory to treatment with other chemotherapeutics due to multidrug resistance.

In a broader sense of the invention, a proliferative disease may furthermore be a hyperproliferative condition, such as leukemias e.g. acute myeloid leukemia, e.g. chronic myeloid leukemia, e.g. chronic lymphatic leukemia, e.g. acute lymphatic leukemia, e.g. multiple myeloma e.g. lymphomas, and/or for use in treatment of myelodysplastic syndrome, systemic mastocytosis, hyperplasias, fibrosis (especially pulmonary, but also other types of fibrosis, such as renal fibrosis), angiogenesis, psoriasis, atherosclerosis, smooth muscle proliferation in the blood vessels, such as stenosis or restenosis following angioplasty, von Hippel-Lindau syndrome, multicentric Castleman disease and/or psioriasis

The combination of the present invention can also be used to prevent or treat diseases that are triggered by persistent angiogenesis, such as psoriasis; Kaposi's sarcoma; restenosis, e.g., stent-induced restenosis; endometriosis; Crohn's disease; Hodgkin's disease; leukemia; arthritis, such as rheumatoid arthritis; hemangioma; angiofibroma; eye diseases, such as diabetic retinopathy and neovascular glaucoma; renal diseases, such as glomerulonephritis; diabetic nephropathy; malignant nephrosclerosis; thrombotic microangiopathic syndromes; transplant rejections and glomerulopathy; fibrotic diseases, such as cirrhosis of the liver; mesangial cell-proliferative diseases; arteriosclerosis; injuries of the nerve tissue; and for inhibiting the re-occlusion of vessels after balloon catheter treatment, for use in vascular prosthetics or after inserting mechanical devices for holding vessels open, such as, e.g., stents, as immunosuppressants, as an aid in scar-free wound healing, and for treating age spots and contact dermatitis.

Combinations of the present invention include use for the treatment, prevention or inhibition of diseases characterized by cell proliferation and infiltration of inflammatory cells such as inflammation, RHA, asthma, chronic bronchitis, artheroschlerosis, and transplant rejection.

Where a tumor, a tumor disease, a carcinoma or a cancer are mentioned, also metastasis in the original organ or tissue and/or in any other location are implied alternatively or in addition, whatever the location of the tumor and/or metastasis.

Suitable clinical studies may be, for example, open label, dose escalation studies in patients with proliferative diseases. Such studies prove in particular the synergism of the active ingredients of the combination of the invention. The beneficial effects on proliferative diseases may be determined directly through the results of these studies which are known as such to a person skilled in the art. Such studies may be, in particular, suitable to compare the effects of a monotherapy using the active ingredients and a combination of the invention. Preferably, the dose of agent (a) is escalated until the Maximum Tolerated Dosage is reached, and agent (b) is administered with a fixed dose. Alternatively, the agent (a) may be administered in a fixed dose and the dose of agent (b) may be escalated. Each patient may receive doses of the agent (a) either daily or intermittent. The efficacy of the treatment may be determined in such studies, e.g., after 12, 18 or 24 weeks by evaluation of symptom scores every 6 weeks.

The administration of a pharmaceutical combination of the invention may result not only in a beneficial effect, e.g. a synergistic therapeutic effect, e.g. with regard to alleviating, delaying progression of or inhibiting the symptoms, but also in further surprising beneficial effects, e.g. fewer side-effects, an improved quality of life or a decreased morbidity, compared with a monotherapy applying only one of the pharmaceutically active ingredients used in the combination of the invention.

A further benefit may be that lower doses of the active ingredients of the combination of the invention may be used, for example, that the dosages need not only often be smaller but may also be applied less frequently, which may diminish the incidence or severity of side-effects. This is in accordance with the desires and requirements of the patients to be treated.

It is one objective of this invention to provide a pharmaceutical combination, such as a composition comprising a quantity of a first component and a second component as previously described, which may be jointly therapeutically effective at targeting or preventing proliferative diseases. These first and second components may be provided for administration in a fixed combination, i.e. in a single galenical composition, which may be prepared in a manner known per se, suitable for enteral, such as oral or rectal, and parenteral administration to mammals (warm-blooded animals), including humans in combination with one or more pharmaceutically acceptable carriers or diluents, especially suitable for enteral or parenteral application.

Alternatively, the combination is a first component and the second component that can be provided as a combination that are separate pharmaceutical doses, including compositions in a kit or pharmaceutical doses not sold as a kit.

The pharmaceutical compositions for separate administration of the first component and the second component may be prepared in a manner known per se and are those suitable for enteral, such as oral or rectal, and parenteral administration to mammals (warm-blooded animals), including humans. Each such composition for separate administration comprises a therapeutically effective amount of at least one pharmacologically active component in combination with one or more pharmaceutically acceptable carriers or diluents.

The term pharmaceutical component is used synonymously as the terms pharmaceutical agent or active ingredient.

Suitable pharmaceutical compositions may contain, for example, from about 0.1 % to about 99.9%, preferably from about 1 % to about 60 %, of the active ingredient(s). Pharmaceutical preparations for the combination therapy for enteral or parenteral administration are, for example, those in unit dosage forms, such as sugar-coated tablets, tablets, capsules or suppositories, or ampoules. If not indicated otherwise, these are prepared in a manner known per se, for example by means of conventional mixing, granulating, sugar-coating, dissolving or lyophilizing processes. It will be appreciated that the unit content of a pharmaceutical component contained in an individual dose of each dosage form need not in itself constitute an effective amount since the necessary effective amount may be reached by administration of a plurality of dosage units.

In a method of treating proliferative disease, the first component and the second component may be administered together, sequentially or separately. The first and second components may be delivered in one combined unit dosage form or in multiple separate unit dosage forms.

In particular, a therapeutically effective amount of each of the pharmaceutical components of the invention may be administered simultaneously or sequentially and in any order, and the components may be administered separately or as a fixed combination. For example, the method of preventing or treating proliferative diseases according to the invention may comprise (i) administration of the first component in free or pharmaceutically acceptable salt form and (ii) administration of the second component in free or pharmaceutically acceptable salt form, simultaneously or sequentially in any order, in jointly therapeutically effective amounts, preferably in synergistically effective amounts, e.g. in daily or intermittently dosages corresponding to the amounts described herein. The individual combination components of the combination of the invention may be administered separately at different times during the course of therapy or concurrently in divided or single combination forms. Furthermore, the term administering also encompasses the use of a prodrug of a combination component that convert *in vivo* to the combination partner as such. The instant invention is therefore to be understood as embracing all such regimens of simultaneous or alternating treatment and the term "administering" is to be interpreted accordingly.

The effective dosage of each of the components employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including the route of administration and the renal and hepatic function of the patient. A clinician or physician of ordinary skill can readily determine and prescribe the effective amount of the single active ingredients required to alleviate, counter or arrest the progress of the condition. Optimal precision in achieving concentration of the active ingredients within the range that yields efficacy without toxicity requires a regimen based on the kinetics of the active ingredients' availability to target sites.

The amount of active ingredient that may be combined with carrier materials to produce a single dosage form will vary depending upon the host treated and the particular mode of administration. It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy. The therapeutically effective amount for a given situation can be readily determined by routine experimentation and is within the skill and judgment of the ordinary clinician.

For purposes of the present invention, a therapeutically effective dose will generally be a total daily dose administered to a host in single or divided doses may be in amounts, for example, of from 0.001 to 1000 mg/kg body weight daily and more preferred from 1.0 to 30 mg/kg body weight daily. Dosage unit compositions may contain such amounts of submultiples thereof to make up the daily dose.

The compounds according to Formula (I), the mTOR inhibitors and the pharmaceutical compositions comprising these active ingredients, may be administered orally, parenterally, sublingually, by aerosolization or inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or ionophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols, which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

The compounds according to Formula (I), mTOR inhibitors and pharmaceutical compositions described herein can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic. Methods to form liposomes are known in the art. See, for example, Prescott (ed.), "Methods in Cell Biology," Volume XIV, Academic Press, New York, 1976, p. 33 *et seq.*

### Example 1

Two cancer derived cell lines are used (BT474 and MDA-MB-231). These are human breast carcinoma cell lines. The cell lines are commercially available from American Type Culture Collection (ATCC). BT474 cells are maintained in Hybri-Care medium (ATCC) supplemented with 10% v/v fetal calf serum and 2 mM L-glutamine. MDB-MB-231 cells are grown in RPMI 1640 medium (Animed, Allschwil, Switzerland) supplemented with 10% v/v fetal calf serum and 2 mM L-glutamine. The media are supplemented with 100 microgramm/ml penicillin/streptomycin and cells are maintained at 37° C in 5% CO₂.

After division and medium change, cells from stock culture are seeded at a density of 3.3 x 10⁴ cells/cm² (BT474) and 1.2 x 10⁴ cells/cm² (MDB-MB-231) on cell plates and incubated for 48 hours at 37° C and 5% CO₂ prior to the treatment with DMSO vehicle, 20 nM everolimus (RAD001) and/or various concentrations of AUY922 (compound 1) for 24 hours. To prepare cell lysates culture plates are washed once with ice-cold phosphor buffered saline (PBS) containing 1 mM phenylmethylsulfonyl fluoride (PMSF) and once with ice-cold extraction buffer (50 mM Hepes pH 7.4, 150 mM NaCl, 25 mM beta-glycerophosphate, 25 mM NaF, 5 mM EGTA, 1 mM EDTA, 15 mM PPi, 2 mM sodium orthovanadate, 10 mM sodium molybdate, leupeptin (10 microgramm/ml), aprotinin (10 microgramm/ml), 1 mM DTT and 1 mM PMSF. Cells are extracted in the same buffer, containing 1% NP-40. The extracts are homogenized, cleared by centrifugation, aliquoted and frozen at -80° C. Protein concentration is determined with the BCA Protein Assay (Pierce, Rockford, IL, USA).

Twenty micrograms of cell extracts are resolved electrophoretically on 12% denaturing sodium dodecyl sulfate polyacrylamide gels (SDS-PAGE) and transferred to polyvinylidene difluoride filters by wet-blotting (1 hour at 250 mA) and probed overnight at 4°C with the following primary antibodies :
anti-phospho-Akt (Ser473) (clone 14-05; 1:2000) obtained from DAKO (Glostrup, Denmark) and diluted in PBS, 0.5 % v/v Tween.
anti-phospho-Akt (T308) (cat # 9275; 1:1000) obtained from Cell Signaling Technology (Beverly, MA, USA) and diluted in PBS, 0.1 % v/v Tween.
anti-Akt (cat # 1085-1; 1:5000) obtained from Epitomics (Burlingame, CA, USA) and diluted in PBS, 0.5 % v/v Tween.

Anti-Actin (cat # MAB1501; 1:20,000) obtained from Chemicon (Billerica, MA, USA) and diluted in PBS, 0.1 % v/v Tween.

After incubation with the appropriate primary antibody (above), decorated proteins are revealed using horseradish peroxidase-conjugated anti-mouse or anti-rabbit immunoglobulins followed by enhanced chemiluminescence (ECL Plus kit) and quantified using Quantity One Software (Bio-Rad, Munich, Germany).

### Example 2

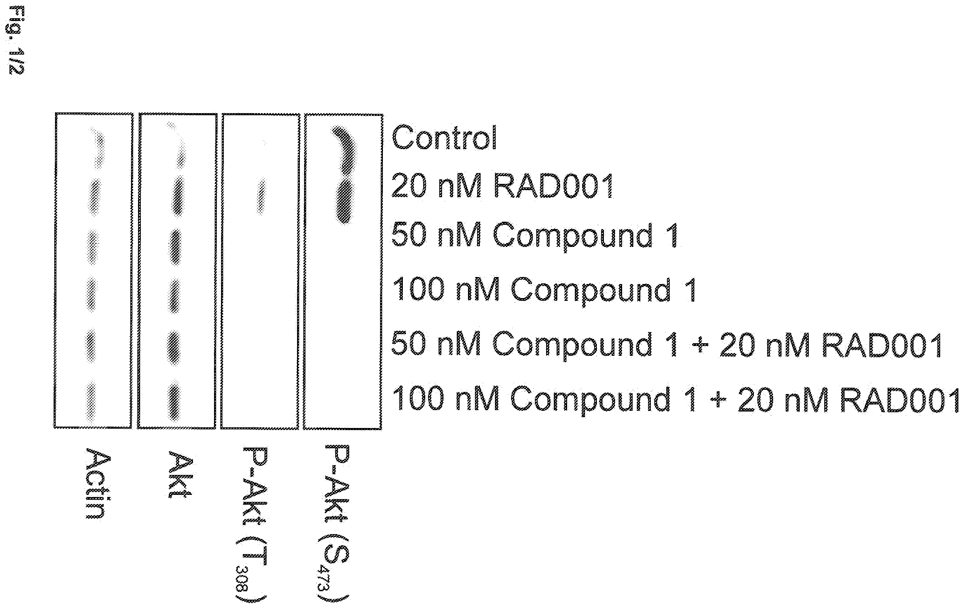

Figure 1 shows AKT phosphorylation levels in presence of everolimus (RAD001) and everolimus (RAD001) in combination with compound I ((R)-2-amino-7-[2-(6-methoxy-pyridin-2-yl)-phenyl]-4-methyl-7,8-dihydro-6H-pyrido[4,3-d]pyrimidin-5-one) in BT474 breast tumor cells. The mTOR inhibition with RAD001 activates Akt in tumor cells (O'Reilly et al., 2006). The Western blot analysis demonstrates that in BT-474 cells treated with 20 nM RAD001 increased levels of phosphorylated AKT (P-AKT(S₄₇₃) and P-AKT(T₃₀₈)) compared to an untreated control are observed. When the cells were treated with 50-100 nM compound I ((R)-2-amino-7-[2-(6-methoxy-pyridin-2-yl)-phenyl]-4-methyl-7,8-dihydro-6H-pyrido[4,3-d]pyrimidin-5-one), AKT phosporylation ((P-AKT(S₄₇₃) and P-AKT(T₃₀₈)) was diminished. In the presence of 50-100 nM compound I, the addition of 20 nM RAD001 did not cause increase in AKT phosphorylation (P-AKT(S₄₇₃) and P-AKT(T₃₀₈)). AKT levels were not significantly affected by any of the treatments. Actin is used to demonstrate equal protein loading on each lane on the Western blot.

### Example 3

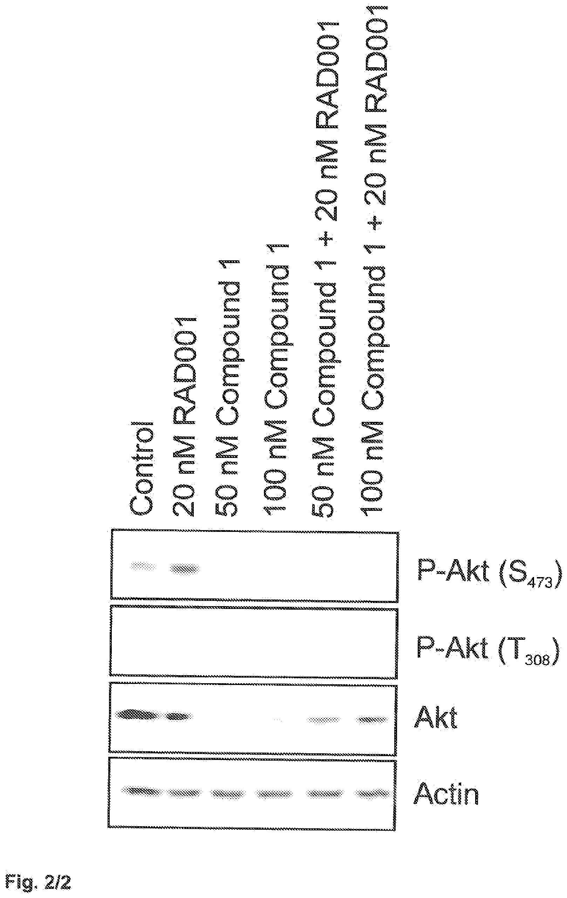

Figure 2 shows the AKT phosphorylation levels in presence of everolimus (RAD001) and everolimus (RAD001) in combination with compound I ((R)-2-amino-7-[2-(6-methoxy-pyridin-2-yl)-phenyl]-4-methyl-7,8-dihydro-6H-pyrido[4,3-d]pyrimidin-5-one) in MDA-MB-231 breast tumor cells. It has been demonstrated that mTOR inhibition with RAD001 activates Akt in tumor cells (O'Reilly et al., 2006). The Western blot analysis demonstrates that in MDA-MB-231 cells treated with 20 nM RAD001 increased levels of phosphorylated AKT (P-AKT(S₄₇₃)) compared to an untreated control are observed. When the cells were treated with 50-100 nM compound I, AKT phosporylation (P-AKT(S₄₇₃)) was diminished. In the presence of 50-100 nM compound I ((R)-2-amino-7-[2-(6-methoxy-pyridin-2-yl)-phenyl]-4-methyl-7,8-dihydro-6H-pyrido[4,3-d]pyrimidin-5-one), addition of 20 nM RAD001 did not cause increased phosphorylation of AKT at amino acid residue T₃₀₈. AKT levels were slightly decreased in the presence of 100 nM Compound I ((R)-2-amino-7-[2-(6-methoxy-pyridin-2-yl)-phenyl]-4-methyl-7,8-dihydro-6H-pyrido[4,3-d]pyrimidin-5-one) consistent with the fact that AKT is a client protein for Hsp90. Actin was used to demonstrate equal protein loading on each lane on the Western blot.

## Claims

1. A pharmaceutical combination comprising (a) a Hsp90 inhibitor which is 5-(2,4-dihydroxy-5-isopropyl-phenyl)-4-(4-morpholin-4-ylmethyl-phenyl)-isoxazole-3-carboxylic acid ethylamide, or a pharmaceutically acceptable salt thereof, and (b) an mTOR inhibitor which is rapamycin, CCI-779, ABT578 or AP23573, or a derivative thereof
according to formula I' 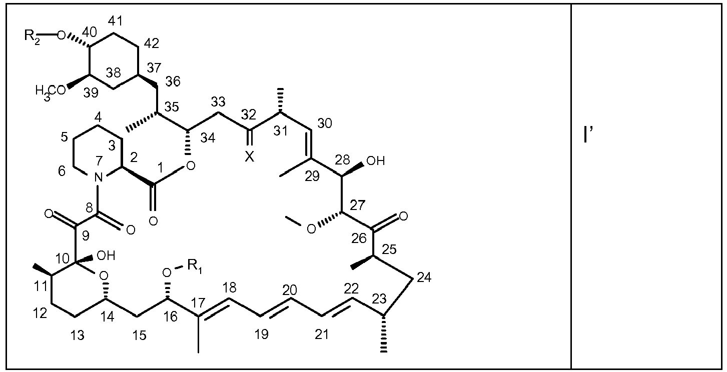 wherein
R₁ is CH₃ or C₃₋₆alkynyl,
R₂ is H or -CH₂-CH₂-OH, 3-hydroxy-2-(hydroxymethyl)-2-methyl-propanoyl or tetrazolyl, and
X is =O, (H,H) or (H,OH),
provided that
R₂ is other than H when X is =O, and
R₁ is CH₃ when R₂ is -CH₂-CH₂-OH.

2. The pharmaceutical combination according to claim 1, wherein the mTOR inhibitor is rapamycin.

3. The pharmaceutical combination according to claim 1, wherein the mTOR inhibitor is everolimus.

4. The pharmaceutical combination according to claim 1, wherein the mTOR inhibitor is CCI-779, ABT578 or AP23573.

5. A first pharmaceutical component in combination with a second pharmaceutical component for use in a method of treating or preventing a proliferative disease, wherein the first pharmaceutical component is a Hsp90 inhibitor which is 5-(2,4-dihydroxy-5-isopropyl-phenyl)-4-(4-morpholin-4-ylmethyl-phenyl)-isoxazole-3-carboxylic acid ethylamide, or a pharmaceutically acceptable salt thereof, and wherein the second pharmaceutical component is an mTOR inhibitor which is as defined in any one of claims 1 to 4.

6. The first pharmaceutical component in combination with a second pharmaceutical component for use according to claim 5 wherein the mTOR inhibitor is everolimus or rapamycin.

7. The first pharmaceutical component in combination with a second pharmaceutical component for use according to claim 5, comprising administering a rapamycin derivative according to formula I' 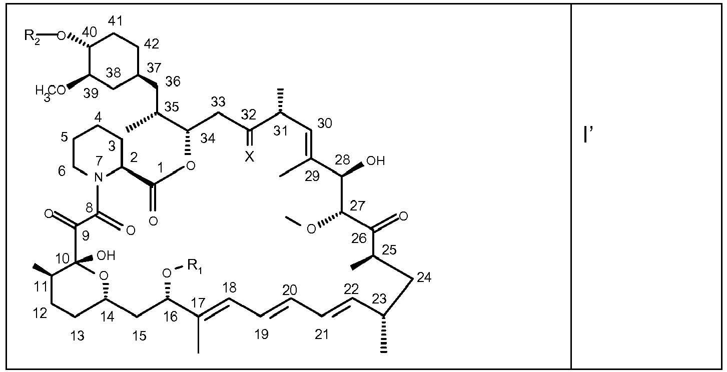 wherein
R₁ is CH₃ or C₃₋₆alkynyl,
X is =O, (H,H) or (H,OH),
R₂ is H or -CH₂-CH₂-OH, 3-hydroxy-2-(hydroxymethyl)-2-methyl-propanoyl or tetrazolyl, and
provided that
R₂ is other than H when X is =O, and
R₁ is CH₃ when R₂ is -CH₂-CH₂-OH.

8. The first pharmaceutical component in combination with a second pharmaceutical component for use according to claim 5, 6 or 7, wherein treating said proliferative disorder is treatment of tumors, myelomas, leukemias, psoriasis, restenosis, sclerodermitis or fibrosis.

9. A kit comprising: (a) a first pharmaceutical composition comprising a Hsp90 inhibitor which is 5-(2,4-dihydroxy-5-isopropyl-phenyl)-4-(4-morpholin-4-ylmethyl-phenyl)-isoxazole-3-carboxylic acid ethylamide, or a pharmaceutically acceptable salt thereof, and (b) an mTOR inhibitor which is as defined in any one of claims 1 to 4, in a second pharmaceutically acceptable carrier.

10. A kit according to claim 9 wherein the mTOR inhibitor is everolimus or rapamycin.

11. The first pharmaceutical component in combination with a second pharmaceutical component for use according to claim 7 or 8, wherein Hsp90 and the mTOR inhibitor are administered simultaneously, concurrently, separately, or sequentially for treating a proliferative disease.

12. A pharmaceutical combination comprising (a) a Hsp90 inhibitor which is 5-(2,4-dihydroxy-5-isopropyl-phenyl)-4-(4-morpholin-4-ylmethyl-phenyl)-isoxazole-3-carboxylic acid ethylamide, or a pharmaceutically acceptable salt thereof, and (b) an mTOR inhibitor which is everolimus for use in a method of treating a proliferative disease which is selected from tumors, myelomas, leukemias, psoriasis, restenosis, sclerodermitis or fibrosis.

## Patentansprüche

1. Pharmazeutische Kombination, umfassend
(a) einen Hsp90-Inhibitor, bei dem es sich um 5-(2,4-Dihydroxy-5-isopropylphenyl)-4-(4-morpholin-4-ylmethylphenyl)isoxazol-3-carbonsäureethylamid oder ein pharmazeutisch unbedenkliches Salz davon handelt, und
(b) einen mTOR-Inhibitor, bei dem es sich um Rapamycin, CCI-779, ABT578 oder AP23573 oder ein Derivat davon
gemäß Formel I' 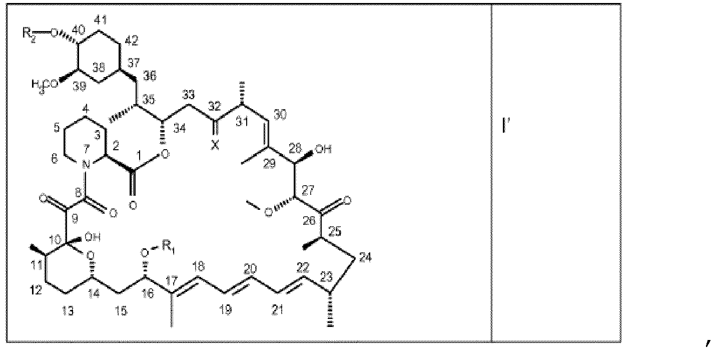 wobei
R₁ für CH₃ oder C₃₋₆-Alkinyl steht,
R₂ für H oder -CH₂-CH₂-OH, 3-Hydroxy-2-(hydroxymethyl)-2-methylpropanoyl oder Tetrazolyl steht und
X für =O, (H,H) oder (H,OH) steht,
mit der Maßgabe, dass
R₂ nicht für H steht, wenn X für =O steht, und
R₁ für CH₃ steht, wenn R₂ für -CH₂-CH₂-OH steht, handelt.

2. Pharmazeutische Kombination nach Anspruch 1, wobei es sich bei dem mTOR-Inhibitor um Rapamycin handelt.

3. Pharmazeutische Kombination nach Anspruch 1, wobei es sich bei dem mTOR-Inhibitor um Everolimus handelt.

4. Pharmazeutische Kombination nach Anspruch 1, wobei es sich bei dem mTOR-Inhibitor um CCI-779, ABT578 oder AP23573 handelt.

5. Erste pharmazeutische Komponente in Kombination mit einer zweiten pharmazeutischen Komponente zur Verwendung in einem Verfahren zur Behandlung oder Prävention einer proliferativen Krankheit, wobei es sich bei der ersten pharmazeutischen Komponente um einen Hsp90-Inhibitor handelt, bei dem es sich um 5-(2,4-Dihydroxy-5-isopropylphenyl)-4-(4-morpholin-4-ylmethylphenyl)isoxazol-3-carbonsäureethylamid oder ein pharmazeutisch unbedenkliches Salz davon handelt, und wobei es sich bei der zweiten pharmazeutischen Komponente um einen mTOR-Inhibitor handelt, welcher wie in einem der Ansprüche 1 bis 4 definiert ist.

6. Erste pharmazeutische Komponente in Kombination mit einer zweiten pharmazeutischen Komponente zur Verwendung nach Anspruch 5, wobei es sich bei dem mTOR-Inhibitor um Everolimus oder Rapamycin handelt.

7. Erste pharmazeutische Komponente in Kombination mit einer zweiten pharmazeutischen Komponente zur Verwendung nach Anspruch 5, umfassend die Verabreichung eines Rapamycinderivats gemäß Formel I' 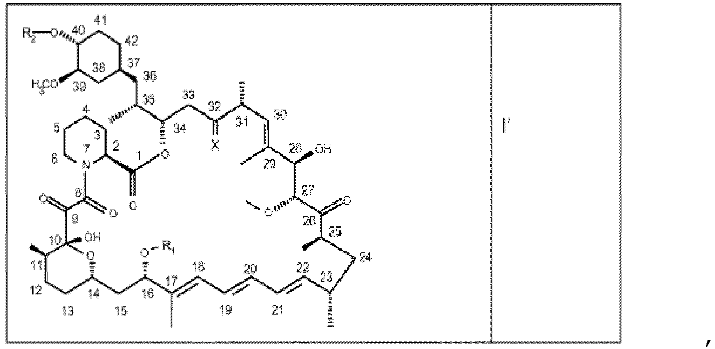 wobei
R₁ für CH₃ oder C₃₋₆-Alkinyl steht,
X für =O, (H,H) oder (H,OH) steht,
R₂ für H oder -CH₂-CH₂-OH, 3-Hydroxy-2-(hydroxymethyl)-2-methylpropanoyl oder Tetrazolyl steht und
mit der Maßgabe, dass
R₂ nicht für H steht, wenn X für =O steht, und
R₁ für CH₃ steht, wenn R₂ für -CH₂-CH₂-OH steht.

8. Erste pharmazeutische Komponente in Kombination mit einer zweiten pharmazeutischen Komponente zur Verwendung nach Anspruch 5, 6 oder 7, wobei es sich bei der Behandlung der proliferativen Erkrankung um eine Behandlung von Tumoren, Myelomen, Leukämien, Psoriasis, Restenose, Sklerodermitis oder Fibrose handelt.

9. Kit, umfassend: (a) eine erste pharmazeutische Zusammensetzung, welche einen Hsp90-Inhibitor umfasst, bei dem es sich um 5-(2,4-Dihydroxy-5-isopropylphenyl)-4-(4-morpholin-4-ylmethylphenyl)isoxazol-3-carbonsäureethylamid oder ein pharmazeutisch unbedenkliches Salz davon handelt, und (b) einen mTOR-Inhibitor, welcher wie in einem der Ansprüche 1 bis 4 definiert ist, in einem zweiten pharmazeutisch unbedenklichen Träger.

10. Kit nach Anspruch 9, wobei es sich bei dem mTOR-Inhibitor um Everolimus oder Rapamycin handelt.

11. Erste pharmazeutische Komponente in Kombination mit einer zweiten pharmazeutischen Komponente zur Verwendung nach Anspruch 7 oder 8, wobei Hsp90 und der mTOR-Inhibitor gleichzeitig, nebeneinander, getrennt oder aufeinanderfolgend zur Behandlung einer proliferativen Krankheit verabreicht werden.

12. Pharmazeutische Kombination, umfassend
(a) einen Hsp90-Inhibitor, bei dem es sich um 5-(2,4-Dihydroxy-5-isopropylphenyl)-4-(4-morpholin-4-ylmethylphenyl)isoxazol-3-carbonsäureethylamid oder ein pharmazeutisch unbedenkliches Salz davon handelt, und
(b) einen mTOR-Inhibitor, bei dem es sich um Everolimus handelt, zur Verwendung in einem Verfahren zur Behandlung einer proliferativen Krankheit ausgewählt aus Tumoren, Myelomen, Leukämien, Psoriasis, Restenose, Sklerodermitis oder Fibrose.

## Revendications

1. Combinaison pharmaceutique comprenant (a) un inhibiteur de Hsp90 qui est l'éthylamide de l'acide 5-(2,4-dihydroxy-5-isopropyl-phényl)-4-(4-morpholin-4-ylméthyl-phényl)-isoxazole-3-carboxylique, ou l'un des sels pharmaceutiquement acceptables de celui-ci, et (b) un inhibiteur de mTOR qui est la rapamycine, CCI-779, ABT578 ou AP23573, ou l'un de leurs dérivés
répondant à la formule I' 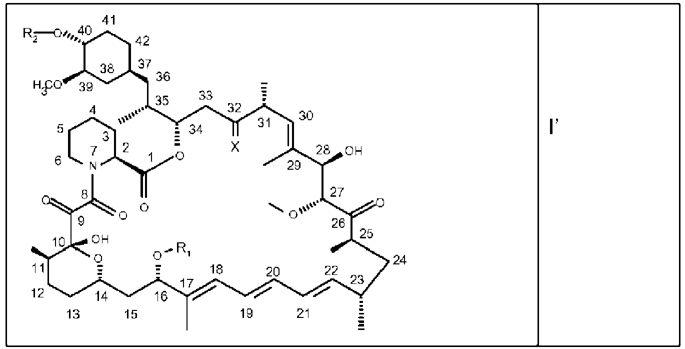 où
R₁ représente un groupement CH₃ ou alcynyle en C₃₋₆,
R₂ représente H ou un groupement -CH₂-CH₂-OH, 3-hydroxy-2-(hydroxyméthyl)-2-méthyl-propanoyle ou tétrazolyle, et
X représente =O, (H,H) ou (H,OH), à la condition que
R₂ soit différent de H lorsque X représente =O, et
R₁ représente CH₃ lorsque R₂ représente -CH₂-CH₂-OH.

2. Combinaison pharmaceutique selon la revendication 1, où l'inhibiteur de mTOR est la rapamycine.

3. Combinaison pharmaceutique selon la revendication 1, où l'inhibiteur de mTOR est l'évérolimus.

4. Combinaison pharmaceutique selon la revendication 1, où l'inhibiteur de mTOR est CCI-779, ABT578 ou AP23573.

5. Premier composant pharmaceutique en combinaison avec un deuxième composant pharmaceutique pour utilisation dans une méthode de traitement prophylactique ou thérapeutique d'une maladie proliférative, où le premier composant pharmaceutique est un inhibiteur de Hsp90 qui est l'éthylamide de l'acide 5-(2,4-dihydroxy-5-isopropyl-phényl)-4-(4-morpholin-4-ylméthyl-phényl)-isoxazole-3-carboxylique, ou l'un des sels pharmaceutiquement acceptables de celui-ci, et où le deuxième composant pharmaceutique est un inhibiteur de mTOR selon l'une quelconque des revendications 1 à 4.

6. Premier composant pharmaceutique en combinaison avec un deuxième composant pharmaceutique pour utilisation selon la revendication 5 où l'inhibiteur de mTOR est l'évérolimus ou la rapamycine.

7. Premier composant pharmaceutique en combinaison avec un deuxième composant pharmaceutique pour utilisation selon la revendication 5, comprenant l'administration d'un dérivé de rapamycine selon la formule I' 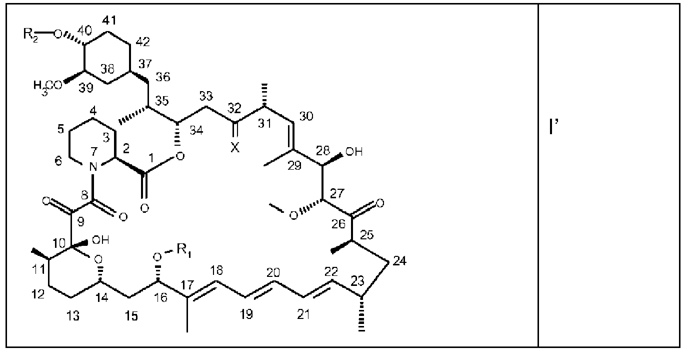 où
R₁ représente un groupement CH₃ ou alcynyle en C₃₋₆,
X représente =O, (H,H) ou (H,OH),
R₂ représente H ou un groupement -CH₂-CH₂-OH, 3-hydroxy-2-(hydroxyméthyl)-2-méthyl-propanoyle ou tétrazolyle, et
à la condition que
R₂ soit différent de H lorsque X représente =O, et
R₁ représente CH₃ lorsque R₂ représente -CH₂-CH₂-OH.

8. Premier composant pharmaceutique en combinaison avec un deuxième composant pharmaceutique pour utilisation selon la revendication 5, 6 ou 7, où le traitement dudit trouble prolifératif est le traitement de tumeurs, de myélomes, de leucémies, du psoriasis, de la resténose, de la sclérodermie ou de la fibrose.

9. Kit comprenant : (a) une première composition pharmaceutique comprenant un inhibiteur de Hsp90 qui est l'éthylamide de l'acide 5-(2,4-dihydroxy-5-isopropyl-phényl)-4-(4-morpholin-4-ylméthyl-phényl)-isoxazole-3-carboxylique, ou l'un des sels pharmaceutiquement acceptables de celui-ci, et (b) un inhibiteur de mTOR selon l'une quelconque des revendications 1 à 4, dans un deuxième vecteur pharmaceutiquement acceptable.

10. Kit selon la revendication 9, où l'inhibiteur de mTOR est l'évérolimus ou la rapamycine.

11. Premier composant pharmaceutique en combinaison avec un deuxième composant pharmaceutique pour utilisation selon la revendication 7 ou 8, où Hsp90 et l'inhibiteur de mTOR sont administrés simultanément, séparément ou séquentiellement dans le traitement d'une maladie proliférative.

12. Combinaison pharmaceutique comprenant (a) un inhibiteur de Hsp90 qui est l'éthylamide de l'acide 5-(2,4-dihydroxy-5-isopropyl-phényl)-4-(4-morpholin-4-ylméthyl-phényl)-isoxazole-3-carboxylique, ou l'un des sels pharmaceutiquement acceptables de celui-ci, et (b) un inhibiteur de mTOR qui est l'évérolimus pour utilisation dans une méthode de traitement d'une maladie proliférative choisie parmi les tumeurs, les myélomes, les leucémies, le psoriasis, la resténose, la sclérodermie et la fibrose.
